# EUROPEAN PATENT APPLICATION

(11) **EP 4 029 948 A1**
(43) Date of publication of application: **20.07.2022**
(21) Application number: 20862190.4
(22) Date of filing: 09.09.2020
(51) Int. Cl.: C12Q 1/26, C12Q 1/28, C12Q 1/30, C12Q 1/34, C12Q 1/44, G01N 33/92

(54) **METHOD AND KIT FOR QUANTIFYING SMALL, DENSE LDL CHOLESTEROL**

(30) Priority: 10.09.2019 JP 2019164403
(71) Applicant: Denka Company Limited, Tokyo 103-8338 (JP)
(72) Inventor: ITOH, Yasuki, Gosen-shi, Niigata 959-1695 (JP); SATOH, Noriyuki, Gosen-shi, Niigata 959-1695 (JP)
(74) Representative: Schrell, Andreas
(86) International application number: PCT/JP2020/034078
(87) International publication number: WO 2021/049518

(57) **Abstract**

This invention provides a method for quantifying cholesterol in small, dense LDL in two steps using an autoanalyzer without pretreatment of an analyte, wherein spontaneous color development of a reagent during storage is suppressed, a kit for quantification used in the method, and a method for preparing such kit. The kit for quantification of cholesterol in small, dense LDL in a sample obtained from a subject used in the method for quantifying cholesterol in small, dense LDL in two steps comprises: (1) a first reagent composition having cholesterol esterase activity, cholesterol oxidase activity, and sphingomyelinase activity and leading cholesterol in lipoproteins other than small, dense LDL to the outside of the reaction system in the presence of cholesterol esterase activity, cholesterol oxidase activity, and sphingomyelinase activity; and (2) a second reagent composition for quantifying cholesterol in small, dense LDL, wherein a coupler, an iron complex, and peroxidase activity are not allowed to be present in the same reagent composition, which is either the first reagent composition or the second reagent composition.

## Description

### Technical Field

The present invention relates to a method and a reagent for measuring cholesterol in small, dense LDL.

### Background Art

Cholesterol is an important constituent of cells and is also a clinically important constituent since an excessive level of cholesterol causes transformation of macrophages into foam cells after uptake thereof by macrophages in the subendothelial space and then causes development of a primary lesion of arteriosclerosis. Low density lipoprotein (LDL) plays a major role in cholesterol transport in the blood and is a risk factor for arteriosclerosis. In particular, cholesterol in small, dense LDL, which is particularly small in particle size among LDLs and higher in density compared with standard LDL, is considered important. When a person exhibits a high cholesterol level in small, dense LDL in the blood, such a person is not visually distinguishable from a healthy person, and it is difficult to identify the current health conditions. However, a risk of causing serious arteriosclerotic diseases, such as heart infarction and apoplectic stroke, 10 years later is increased for such a person. In order to predict a risk of causing serious arteriosclerotic diseases in the future, accordingly, it is very important to perform fractional measurement of cholesterol in small, dense LDL.

Examples of conventional methods for measurement of small, dense LDL include an ultracentrifugation method, an electrophoresis method, and a method using high performance liquid chromatography. These methods are not convenient since they require expensive facilities and much time for measurement. While methods of counting the number of LDL particles or the number of sd LDL-C particles by NMR are available, such methods require the use of NMR apparatuses, and it is accordingly difficult to perform such methods in general hospital laboratories or medical examination centers.

An example of a method for measuring small, dense LDL using an autoanalyzer is a method (Patent Document 1) that involves suspending or dissolving small particle LDL with the use of differences in ionic strength and then conducting measurement on the small particle LDL with the use of differences in absorbance. According to such method, however, differences in absorbance are measured based on turbidity. Thus, it is impossible to measure cholesterol in small, dense LDL, and specificity and accuracy are insufficient. Also, a method that involves measuring cholesterol or triglyceride in small, dense LDL with the use of a separating agent comprising polyanions and a divalent cation in combination with a reagent adaptable for an autoanalyzer is known (Patent Document 2). According to this method, lipid components in small, dense LDL can be more easily measured compared with ultracentrifugation or electrophoresis, and this method is excellent in terms of specificity and accuracy. This method, however, requires pretreatment of specimens and a procedure for separating LDL into small, dense LDL and LDL other than such LDL.

Examples of methods for measuring small, dense LDL using an autoanalyzer include: a method for quantifying cholesterol in small, dense LDL remaining in a reaction solution comprising a step of eliminating cholesterol in lipoproteins other than small, dense LDL in a reaction solution that inhibits the reaction of cholesterol in small, dense LDL (Patent Document 3); a method for quantifying cholesterol comprising a first step of eliminating cholesterol in LDL other than small, dense LDL in the presence of sphingomyelinase and quantifying cholesterol in lipoproteins remaining after the first step (Patent Document 4); and a method for quantifying cholesterol in small, dense LDL in a sample obtained from a subject comprising: a first step of eliminating lipoproteins other than small, dense LDLs in a sample in the presence of cholesterol esterase and 0.05 g/l to 1.0g/l of a surfactant that acts on the lipoproteins other than small, dense LDLs; and a second step of quantifying cholesterol in small, dense LDLs remaining after the first step (Patent Document 5).

An example of a kit used for measurement of cholesterol in small, dense LDL is a reagent composition comprising sphingomyelinase used in the first step, a surfactant used in the second step, which is a polyoxyethylene-polyoxypropylene copolymer, a derivative thereof, or a polyoxyethylene derivative with HLB of 11 to less than 14, 4-aminoantipyrine, and peroxidase (Patent Document 6).

According to such methods for quantifying cholesterol in small, dense LDL using an autoanalyzer without performing pretreatment of specimens, however, the second reagent would spontaneously become yellow with the elapse of time, and storage stability of the reagent was an issue of concern.

### Prior Art Documents

### Patent Documents

Patent Document 1: JP 2003-28882 A
Patent Document 2: WO 2004/053500
Patent Document 3: JP Patent No. 5111389
Patent Document 4: JP Patent No. 5450080
Patent Document 5: JP Patent No. 5325093
Patent Document 6: JP Patent No. 5450080

### Summary of the Invention

### Objects to Be Attained by the Invention

The present invention provides a method for quantifying cholesterol in small, dense LDL using an autoanalyzer without performing pretreatment of specimens in two steps, a method for suppressing spontaneous color development of a reagent during storage in the method of quantification, a kit for quantification used in the method of quantification, and a method for preparing such kit.

### Means for Attaining the Objects

The present inventors have conducted concentrated studies. As a result, they discovered that spontaneous color development of a reagent would be suppressed and reagent stability would be improved by regulating a combination of components such as an enzyme, an electron donor, a coupler, and an iron complex, to be contained in a first reagent used in a first step and a combination thereof to be contained in a second reagent used in a second step.

Specifically, they discovered that spontaneous color development of a reagent would be suppressed by refraining from allowing a coupler, an iron complex, and peroxidase to be present in the same reagent composition, which is either of two reagent compositions; i.e., a first reagent composition for eliminating cholesterol in LDL other than small, dense LDL and a second reagent composition for quantifying cholesterol in small, dense LDL, and, in particular, by allowing the coupler and the iron complex to be separately present in such 2 reagent compositions. This has led to the completion of the present invention.

Specifically, the present invention is as described below.
[1] A kit for quantification of cholesterol in small, dense LDL in a sample obtained from a subject used in a method for quantifying cholesterol in small, dense LDL in two steps comprising:
   (1) a first reagent composition having cholesterol esterase activity, cholesterol oxidase activity, and sphingomyelinase activity and leading cholesterol in lipoproteins other than small, dense LDL to the outside of the reaction system in the presence of cholesterol esterase activity, cholesterol oxidase activity, and sphingomyelinase activity; and
   (2) a second reagent composition for quantifying cholesterol in small, dense LDL,
   wherein a coupler, an iron complex, and peroxidase are not allowed to be present in the same reagent composition, which is either the first reagent composition or the second reagent composition.
[2] A kit for quantification of small, dense LDL cholesterol in a sample obtained from a subject used in a method for quantifying cholesterol in small, dense LDL in two steps comprising:
   (1) a first reagent composition having cholesterol esterase activity, cholesterol oxidase activity, and sphingomyelinase activity and leading cholesterol in lipoproteins other than small, dense LDL to the outside of the reaction system in the presence of cholesterol esterase activity, cholesterol oxidase activity, and sphingomyelinase activity; and
   (2) a second reagent composition for quantifying cholesterol in small, dense LDL,
   wherein a coupler and an iron complex are not allowed to be present in the same reagent composition, which is either the first reagent composition or the second reagent composition.
[3] The kit according to [1] or [2], wherein the first reagent composition comprises catalase activity and a coupler and the second reagent composition comprises an electron donor, an iron complex, and peroxidase activity.
[4] The kit according to [1] or [2], wherein the first reagent composition comprises peroxidase activity and a coupler and the second reagent composition comprises an electron donor and an iron complex.
[5] The kit according to [1], wherein the first reagent composition comprises catalase activity, a coupler, and an iron complex and the second reagent composition comprises an electron donor and peroxidase activity.
[6] The kit according to [1], wherein the first reagent composition comprises peroxidase activity and an electron donor and the second reagent composition comprises a coupler and an iron complex.
[7] The kit according to [1] or [2], wherein the first reagent composition comprises catalase activity, an electron donor, and an iron complex and the second reagent composition comprises peroxidase activity and a coupler.
[8] The kit according to [1] or [2], wherein the first reagent composition comprises peroxidase activity, an electron donor, and an iron complex and the second reagent composition comprises a coupler.
[9] The kit according to any of [4], [6], and [8], wherein the first reagent composition further comprises catalase activity.
[10] The kit according to any of [1] to [9], wherein the first reagent composition further comprises a surfactant that acts on lipoproteins other than small, dense LDL and the second reagent composition further comprises a surfactant that acts at least on small, dense LDL.
[11] The kit according to [10], wherein the surfactant contained in the first reagent composition comprises polyoxyethylene polycyclic phenyl ether.
[12] The kit according to [11], wherein the polyoxyethylene polycyclic phenyl ether includes a polyoxyethylene benzyl phenyl ether derivative and/or a polyoxyethylene styrenated phenyl ether derivative.
[13] A method for quantifying cholesterol in small, dense LDL in a sample obtained from a subject in two steps comprising:
   (1) a first step of leading cholesterol in lipoproteins other than small, dense LDL to the outside of the reaction system in the presence of cholesterol esterase activity, cholesterol oxidase activity, and sphingomyelinase activity; and
   (2) a second step of quantifying cholesterol in lipoproteins remaining after the first step,
   wherein, in either the step (1) or (2), the coupler, the iron complex, and peroxidase are not used simultaneously.
[14] A method for quantifying cholesterol in small, dense LDL in a sample obtained from a subject in two steps comprising:
   (1) a first step of leading cholesterol in lipoproteins other than small, dense LDL to the outside of the reaction system in the presence of cholesterol esterase activity, cholesterol oxidase activity, and sphingomyelinase activity; and
   (2) a second step of quantifying cholesterol in lipoproteins remaining after the first step,
   wherein, in either the step (1) or (2), the coupler and the iron complex are not used simultaneously.
[15] The method of quantification according to [13] or [14], wherein the first step involves the use of catalase activity and a coupler and the second step involves the use of an electron donor, an iron complex, and peroxidase activity.
[16] The method of quantification according to [13] or [14], wherein the first step involves the use of peroxidase activity and a coupler and the second step involves the use of an electron donor and an iron complex.
[17] The method of quantification according to [13], wherein the first step involves the use of catalase activity, a coupler, and an iron complex and the second step involves the use of an electron donor and peroxidase activity.
[18] The method of quantification according to [13], wherein the first step involves the use of peroxidase activity and an electron donor and the second step involves the use of a coupler and an iron complex.
[19] The method of quantification according to [13] or [14], wherein the first step involves the use of catalase activity, an electron donor, and an iron complex and the second step involves the use of peroxidase activity and a coupler.
[20] The method of quantification according to [13] or [14], wherein the first step involves the use of peroxidase activity, an electron donor, and an iron complex and the second step involves the use of a coupler.
[21] The method of quantification according to any of [14], [18], and [20], wherein the first step further involves the use of catalase activity.
[22] The method of quantification according to any of [13] to [21], wherein the first step further involves the use of a surfactant that acts on lipoproteins other than small, dense LDL and the second step further involves the use of a surfactant that acts at least on small, dense LDL.
[23] Use of a first reagent composition and a second reagent composition for producing a kit for quantification of small, dense LDL cholesterol in a sample obtained from a subject in the method for quantifying cholesterol in small, dense LDL in two steps:
   (1) a first reagent composition having cholesterol esterase activity, cholesterol oxidase activity, and sphingomyelinase activity; and
   (2) a second reagent composition for quantifying cholesterol in small, dense LDL,
   wherein a coupler, an iron complex, and peroxidase are not allowed to be present in the same reagent composition, which is either the first reagent composition or the second reagent composition.
[24] Use of a first reagent composition and a second reagent composition for producing a kit for quantification of small, dense LDL cholesterol in a sample obtained from a subject in the method for quantifying cholesterol in small, dense LDL in two steps:
   (1) a first reagent composition having cholesterol esterase activity, cholesterol oxidase activity, and sphingomyelinase activity; and
   (2) a second reagent composition for quantifying cholesterol in small, dense LDL,
   wherein a coupler and an iron complex are not allowed to be present in the same reagent composition, which is either the first reagent composition or the second reagent composition.

This description includes part or all of the content as disclosed in the description and/or drawings of Japanese Patent Application No. 2019-164403, which is a priority document of the present application.

### Effects of the Invention

The present invention provides a method for stable quantification of cholesterol in small, dense LDL with improved stability while suppressing spontaneous color development of 2 reagents during storage used when subjecting cholesterol in small, dense LDL to fractional measurement separately from other cholesterols, a kit for quantification used in the method of quantification, and a method for producing such kit used to implement the method of quantification.

### Brief Description of the Drawings

Fig. 1 shows absorption spectra immediately after preparation and after storage of the second reagent composition in Comparative Example 1.
Fig. 2 shows absorption spectra immediately after preparation and after storage of the first reagent composition in Comparative Example 2.
Fig. 3a shows absorption spectra immediately after preparation and after storage of the first reagent composition in Example 1.
Fig. 3b shows absorption spectra immediately after preparation and after storage of the second reagent composition in Example 1.
Fig. 4a shows absorption spectra immediately after preparation and after storage of the first reagent composition in Example 2.
Fig. 4b shows absorption spectra immediately after preparation and after storage of the second reagent composition in Example 2.
Fig. 5a shows absorption spectra immediately after preparation and after storage of the first reagent composition in Example 3.
Fig. 5b shows absorption spectra immediately after preparation and after storage of the second reagent composition in Example 3.
Fig. 6a shows absorption spectra immediately after preparation and after storage of the first reagent composition in Example 4.
Fig. 6b shows absorption spectra immediately after preparation and after storage of the second reagent composition in Example 4.
Fig. 7a shows absorption spectra immediately after preparation and after storage of the first reagent composition in Example 5.
Fig. 7b shows absorption spectra immediately after preparation and after storage of the second reagent composition in Example 5.
Fig. 8a shows absorption spectra immediately after preparation and after storage of the first reagent composition in Example 6.
Fig. 8b shows absorption spectra immediately after preparation and after storage of the second reagent composition in Example 6.
Fig. 9a shows absorption spectra immediately after preparation and after storage of the first reagent composition in Example 7.
Fig. 9b shows absorption spectra immediately after preparation and after storage of the second reagent composition in Example 7.
Fig. 10a shows absorption spectra immediately after preparation and after storage of the first reagent composition in Example 8.
Fig. 10b shows absorption spectra immediately after preparation and after storage of the second reagent composition in Example 8.
Fig. 11a shows absorption spectra immediately after preparation and after storage of the first reagent composition in Example 9.
Fig. 11b shows absorption spectra immediately after preparation and after storage of the second reagent composition in Example 9.

### Embodiments of the Invention

Hereafter, the present invention is described in detail.

Lipoproteins can be fractionated roughly into VLDL, LDL, and HDL. LDL is further fractionated into small, dense LDL and other sub-fractions. Small, dense LDL is also referred to as small particle LDL, SLDL (small LDL), dense LDL, or sd LDL. Other LDL may also be referred to as L LDL (large LDL) or light LDL. These fractions may be distinguished from sub-fractions based on particle size or density. The particle size (or particle diameter) of VLDL ranges from 30 nm to 80 nm (34 nm to 75 nm), that of LDL ranges from 22 nm to 28 nm (19 nm to 30 nm), and that of HDL ranges from 7 nm to 10 nm, although such figures may vary depending on researchers. The density of VLDL is 1.006 (g/1) or less, that of LDL ranges from 1.019 to 1.063 (g/1), and that of HDL ranges from 1.063 to 1.21 (g/1). The diameters of LDL particles can be measured by gradient gel electrophoresis (GGE) (JAMA, 260, pp. 1917-21, 1988) or NMR (HANDBOOK OF LIPOPROTEIN TESTING 2nd Edition, Edited by Nader Rifai et al., pp. 609-623, AACC PRESS: The Fats of Life, Summer 2002, LVDD 15 YEAR ANNIVERSARY ISSUE, Volume AVI No. 3, pp. 15-16). Density can be determined based on analyses by ultracentrifugation (Atherosclerosis, 106, pp. 241-253, 1994: Atherosclerosis, 83, p. 59, 1990).

Small, dense LDL fractions to be measured by the method of the present invention are generally sub-fractions with diameters ranging from approximately 22.0 nm to approximately 25.5 nm and density ranging from 1.040 to 1.063 (g/1), among LDL fractions. The reason why LDL is sub-fractionated based on particle size is that small LDL among LDL needs to be fractionally measured because such LDL with small particle sizes has a high tendency of inducing arteriosclerosis and has particularly high malignancy among LDLs. The distributions of diameter and density of LDL are continuous. Thus, it is impossible to clearly determine that an LDL with a density that is at the aforementioned level or higher results in a particularly high degree of malignancy. Thus, the density ranging from 1.040 to 1.063 (g/1) does not constitute the established characteristics of small, dense LDL, but is a value obtained by dividing the density range between 1.019 and 1.063 (g/1) at the center point, which is a process extensively used and well established. In another report, for example, small, dense LDL is fractionated in the range between 1.044 and 1.060 (g/1) (Atherosclerosis: 106 241-253 1994). There are some differences among researchers on how to set the range of density for small, dense LDL. In all cases, the presence of small, dense LDL is associated with clinical malignancy when fractionation is performed using such density range.

In the present invention, the term "small, dense LDL" refers to LDL that has a low density among LDL and clinically has a higher tendency of inducing arteriosclerosis than other LDL. Preferably, small, dense LDL has a density higher than the center point within the entire density range of LDL. More preferably, small, dense LDL has a density within the range between 1.040 and 1.063 (g/1). Also, the term "lipoproteins other than LDL" refers to VLDL and HDL. This term may further refer to chylomicron, IDL (intermediate density lipoprotein) and VHDL (very high density lipoprotein).

The quantification kit of the present invention is used in the method for quantifying cholesterol in small, dense LDL in two steps. The first reagent composition of the quantification kit of the present invention is used in the first step and the second reagent composition thereof is used in the second step.

The first reagent composition of the kit for quantifying cholesterol in small, dense LDL according to the present invention has cholesterol esterase, cholesterol oxidase, sphingomyelinase and leading cholesterol in lipoproteins other than small, dense LDL to the outside of the reaction system in the presence of cholesterol esterase activity, cholesterol oxidase activity, and sphingomyelinase activity.

Examples of ingredients of the first reagent composition include, but are not limited to, albumin, a buffer, a surfactant, catalase, peroxidase, a surfactant comprising a polyoxyethylene derivative, and an electron donor.

The second reagent composition in the kit for quantification of cholesterol in small, dense LDL according to the present invention comprises ingredients for quantification of cholesterol in small, dense LDL. While ingredients of the second reagent composition vary depending on the first reagent composition, a known substance can be used without particular limitation, provided that cholesterol in small, dense LDL can be quantified.

Examples of ingredients of the second reagent composition include, but are not limited to, peroxidase, a polyoxyethylene derivative, and a coupler.

The method of the present invention comprises 2 steps. In the first step, a surfactant that acts on LDLs other than small, dense LDL (hereafter, may be referred to as Large LDL or L LDL) or other lipoproteins, such as VLDL or HDL, is allowed to react with a sample obtained from a subject in the presence of sphingomyelinase and cholesterol esterase, and cholesterol generated upon liberation from lipoproteins is allowed to react with cholesterol oxidase and led to the outside of the reaction system. In the subsequent second step, cholesterol in small, dense LDL remaining unreacted after the first step is quantified.

The method of the present invention is generally carried out within an autoanalyzer.

In the method of the present invention, an electron donor is subjected to coupling with a coupler in the presence of peroxidase activity when quantifying the cholesterol level in small, dense LDL, and the absorbance at a particular wavelength is measured based on the generated dye. In such a case, an iron complex is used as a reaction promoter. When all of the coupler, peroxidase activity, and the iron complex are present in the same reagent, however, the reagent would undergo spontaneous color development with the elapse of time, and quantification of small, dense LDL cholesterol would be adversely affected. In the present invention, accordingly, either of the coupler, peroxidase activity, and the iron complex was allowed to be present in another reagent, so that the reagent could be prevented from undergoing spontaneous color development and stabilized. When "peroxidase activity is present," peroxidase is present, and a reaction catalyzed by peroxidase could occur. The expression "peroxidase activity is present" can be understood as "peroxidase is present." The same applies to cholesterol esterase activity, cholesterol oxidase activity, catalase activity, and sphingomyelinase activity.

In the method of the present invention, the coupler, the peroxidase activity, and the iron complex are allowed to be present in the first reagent composition used in the first step and the second reagent composition used in the second step separately from each other as described below.
(1) The coupler is allowed to be present in the first reagent composition used in the first step, and the peroxidase activity and the iron complex are allowed to be present in the second reagent composition used in the second step.
(2) The coupler and the peroxidase activity are allowed to be present in the first reagent composition and the iron complex is allowed to be present in the second reagent composition.
(3) The peroxidase activity is allowed to be present in the first reagent composition and the coupler and the iron complex are allowed to be present in the second reagent composition.
(4) The coupler and the iron complex are allowed to be present in the first reagent composition and the peroxidase activity is allowed to be present in the second reagent composition.
(5) The peroxidase activity and the iron complex are allowed to be present in the first reagent composition and the coupler is allowed to be present in the second reagent composition.
(6) The iron complex is allowed to be present in the first reagent composition and peroxidase activity and the coupler are allowed to be present in the second reagent composition.

In addition, the coupler and the electron donor are preferably allowed to be present in different reagent compositions separately from each other.

Hereafter, each step is described in detail.

The kit of the present invention comprises a first reagent composition used in a step of leading cholesterol in lipoproteins other than small, dense LDL to the outside of the reaction system and a second reagent composition used in a step of measuring cholesterol in small, dense LDL.

In the step of leading cholesterol in lipoproteins other than small, dense LDL to the outside of the reaction system, conventional techniques, such as a technique of eliminating cholesterol in lipoproteins other than small, dense LDL and leading such cholesterol to the outside of the reaction system and a technique of allowing cholesterol in lipoproteins other than small, dense LDL to aggregate or inhibiting such cholesterol from undergoing a reaction in the subsequent step, can be employed.

The first step of eliminating cholesterol in lipoproteins other than small, dense LDL and leading such cholesterol to the outside of the reaction system is carried out in the manner described below:
(1) a method of generating hydrogen peroxide from cholesterol in lipoproteins with the aid of cholesterol esterase activity and cholesterol oxidase activity and degrading hydrogen peroxide into water and oxygen in the presence of catalase activity;
(2) a method of forming colorless quinone in the presence of hydrogen peroxide generated with the aid of cholesterol esterase activity and cholesterol oxidase activity and a coupler or an electron donor; or
(3) a method of degrading hydrogen peroxide in the presence of catalase activity and, at the same time, forming colorless quinone in the presence of a coupler or an electron donor.

In the method (1) of degrading hydrogen peroxide in the presence of catalase activity, it is necessary that catalase activity is present in the first reagent composition used in the first step. In the method (2) of forming colorless quinone, in addition, it is necessary that at least one of a coupler and an electron donor is present in the first reagent composition and that peroxidase activity is further present in the first reagent composition. In the method (3) of degrading hydrogen peroxide with the aid of catalase activity and, at the same time, forming colorless quinone in the presence of a coupler or an electron donor, it is necessary that catalase activity, peroxidase activity, and a coupler or an electron donor are present in the first reagent composition.

Examples of couplers that can be used in the coupling reaction in the present invention include, but are not limited to, 4-aminoantipyrine, aminoantipyrine derivative, vanillin diamine sulfonic acid, methylbenzothiazolinone hydrazone, and sulfonylated methylbenzothiazolinone hydrazone.

An electron donor that is preferably used in the present invention is an aniline derivative. Examples of such aniline derivative include N-ethyl-N-(2-hydroxy-3-sulfopropyl)-3-methylaniline (TOOS), N-ethyl-N-(2-hydroxy-3-sulfopropyl)-3,5-dimethylaniline (MAOS), N-ethyl-N-(3-sulfopropyl)-3-methylaniline (TOPS), N-(2-hydroxy-3-sulfopropyl)-3,5-dimethoxyaniline (HDAOS), N-(3-sulfopropyl)aniline (HALPS), and N-(3-sulfopropyl)-3-methoxy-5-aniline (HMMPS). The final concentration of such electron donor to be used in a reaction solution preferably ranges from 0.1 mmol/1 to 8 mmol/l.

Examples of iron complexes used in the present invention include potassium ferrocyanide, sodium ferrocyanide, a porphyrin-iron complex, and an EDTA-iron complex. A preferable concentration of the iron complex is 0.001 to 0.05 mmol/l.

In the method of the present invention, a step of eliminating cholesterol in lipoproteins other than small, dense LDL and leading such cholesterol to the outside of the reaction system involves the use of a surfactant comprising sphingomyelinase and a polyoxyethylene derivative. In the present invention, in addition, the concentration of the sphingomyelinase activity in the reagent is preferably 0.1 to 100 U/ml and more preferably 0.2 to 20 U/ml. The concentration in the reaction solution during the reaction is preferably 0.05 to 100 U/ml, and more preferably 0.1 to 20 U/ml. Specific examples of preferable sphingomyelinase include, but are not limited to, SPC (Asahi Kasei Corporation), Sphingomyelinase from *Bacillus cereus*, and Sphingomyelinase from *Staphylococcus aureus* (SIGMA).

In the step of eliminating cholesterol in lipoproteins other than small, dense LDL, such as L LDL, VLDL, and HDL, cholesterol in lipoproteins other than small, dense LDL is eliminated in the presence of a surfactant that acts on lipoproteins other than small, dense LDL but does not react with small, dense LDL. An example of a surfactant that acts on and reacts with lipoproteins other than small, dense LDL is a polyoxyethylene derivative. Examples of derivatives include nonionic surfactants selected from among polyoxyethylene alkyl ether and polyoxyethylene polycyclic phenyl ether. Preferable examples of polyoxyethylene polycyclic phenyl ether include a polyoxyethylene benzylphenyl derivative, a polyoxyethylene styrenated phenyl ether derivative, and specific phenol ethoxylate. Specific examples of polyoxyethylene polycyclic phenyl ether include: EMULGEN A-60, EMULGEN A-500, EMULGEN B-66, and EMULGEN A-90 (Kao Corporation); Newcol 703, Newcol 704, Newcol 706, Newcol 707, Newcol 708, Newcol 709, Newcol 710, Newcol 711, Newcol 712, Newcol 714, Newcol 719, Newcol 723, Newcol 729, Newcol 733, Newcol 740, Newcol 747, Newcol 780, Newcol 610, Newcol 2604, Newcol 2607, Newcol 2609, and Newcol 2614 (NIPPON NYUKAZAI CO., LTD.); Noigen EA-87, Noigen EA-137, Noigen EA-157, Noigen EA-167, Noigen EA-177, Noigen EA-197D, and Noigen EA-207D (Dai-ichi Kogyo Seiyaku); and Blaunon DSP-9, Blaunon DSP-12.5, Blaunon TSP-7.5, Blaunon TSP-16, and Blaunon TSP-50 (Aoki Oil Industrial Co., Ltd.). The concentration of a surfactant in a reagent is preferably 0.3% to 5% (w/v), and more preferably 0.5% to 3% (w/v). The concentration thereof in a reaction solution during the reaction is preferably 0.15% to 5%, and more preferably 0.25% to 3% (w/v). A surfactant in a reagent can be identified by a method of analysis comprising performing, for example, IR, NMR, and LC-MS in adequate combination. Examples of methods of identifying the ionic property of a surfactant (i.e., nonionic, anionic, or cationic property) include an extraction method and a solid-phase extraction method using an organic solvent under acidic or alkaline conditions. A surfactant structure can be analyzed by LC-MSMS or NMR.

In the above step of eliminating cholesterol in lipoproteins other than small, dense LDL, for example, cholesterol esterase acts on L LDL in the presence of sphingomyelinase and a surfactant that acts on lipoproteins other than small, dense LDL, and the resulting cholesterol is allowed to react and eliminated in the presence of an enzyme that acts with cholesterol, such as cholesterol oxidase. Thus, cholesterol in lipoproteins is led to the outside of the reaction system. When "a surfactant acts on (reacts with)," a surfactant degrades lipoproteins and liberates cholesterol in lipoproteins. For example, "a surfactant that acts on (reacts with) lipoproteins other than small, dense LDL" is not required to be completely unreactive with small, dense LDL, but it may be mainly reactive with lipoproteins other than small, dense LDL. The term "elimination" means to degrade a substance in a sample obtained from a subject, so as to prevent the degraded product from being detected in the subsequent step. When "eliminating cholesterol in lipoproteins other than small, dense LDL," specifically, lipoproteins other than small, dense LDL in a sample obtained from a subject are degraded, and the degraded product; that is, cholesterol in lipoproteins, is prevented from being detected in the subsequent step.

In the present invention, the phrase "led (or ..leading...) to the outside of the reaction system" refers to a process in which cholesterol contained in HDL, VLDL, L LDL, or the like is eliminated, aggregated, or inhibited to avoid its reaction in the subsequent steps, for example, in order to prevent such cholesterol contained in HDL, VLDL, L LDL, or the like from affecting quantification of small, dense LDL cholesterol.

Cholesterol esterase and cholesterol oxidase are caused to act in the presence of the above surfactant to generate hydrogen peroxide from cholesterol and then the thus generated hydrogen peroxide is eliminated. The concentration of cholesterol esterase in a reaction solution preferably ranges from approximately 0.010 U/ml to 10 U/ml. Also, cholesterol oxidase derived from a bacterium or yeast can be used. The concentration of cholesterol oxidase in a reaction solution preferably ranges from approximately 0.1 U/ml to 0.7 U/ml. The concentration of catalase in a reaction solution preferably ranges from approximately 40 U/ml to 2500 U/ml. The concentration of peroxidase in a reaction solution when converting hydrogen peroxide into colorless quinone preferably ranges from 0.4 U/ml to 2.0 U/ml

In the step of eliminating cholesterol in lipoproteins other than small, dense LDL, cholesterol in the lipoproteins that had acted on and reacted with the surfactant and cholesterol esterase can be allowed to react with a cholesterol-reactive enzyme, such as cholesterol oxidase, and led to the outside of the reaction system. Cholesterol in lipoproteins other than L LDL or LDL, such as VLDL or HDL, can be led to the outside of the reaction system through the reaction as described above. In the subsequent step, accordingly, small, dense LDLs would selectively remain as lipoproteins in the reaction solution. In the present invention, lipoproteins other than small, dense LDL are eliminated and led to the outside of the reaction system, so as to prevent cholesterol in lipoproteins other than small, dense LDL from being detected in the subsequent step. This process may be referred to as "differentiation of small, dense LDL from other lipoproteins."

In the step of eliminating cholesterol in lipoproteins other than small, dense LDL, cholesterol in lipoproteins other than small, dense LDL is not necessarily completely eliminated. In such a case, a surfactant that enables selective measurement of cholesterol in small, dense LDL may be used in a step of quantifying cholesterol in small, dense LDL.

In the step of eliminating cholesterol in lipoproteins other than small, dense LDL and leading such cholesterol to the outside of the reaction system, the concentration of cholesterol esterase in the reaction solution is preferably 0.01 U/ml to 10.0 U/ml, more preferably 0.3 U/ml to 2.5 U/ml, and particularly preferably 0.6 U/ml to 2.0 U/ml. Cholesterol esterase used in the present invention is not particularly limited, provided that such enzyme can hydrolyze cholesterol ester. Cholesterol esterase derived from animal- or microbe can be used.

A reaction solution used in the step of eliminating cholesterol in lipoproteins other than small, dense LDL can be optionally supplemented with a lipoprotein-degrading enzyme, so as to regulate the activity on various lipoproteins. An example of a lipoprotein-degrading enzyme that can be used is lipoprotein lipase. Lipoprotein lipase is not particularly limited, provided that such enzyme can degrade a lipoprotein. Lipoprotein lipase derived from animal- or microbe can be used. The concentration of lipoprotein lipase in the reaction solution is preferably 0.01 U/ml to 10 U/ml, more preferably 0.01 U/ml to 5 U/ml, and particularly preferably 0.01 U/ml to 1 U/ml.

In the present invention, cholesterol in small, dense LDL that has remained unreacted in the step of leading cholesterol in lipoproteins other than small, dense LDL is quantified in the subsequent second step. A conventional method for LDL quantification can be used in the step of quantification. Examples of methods include a method that involves adding an LDL coagulant and then quantifying the content of the thus formed LDL specific aggregates by turbidimetric determination, a method that involves using an antigen-antibody reaction with an LDL specific antibody, and a method that involves quantifying degraded products using enzymes. Of these methods, a method involving quantification of degraded products using an enzyme is preferable.

In such method, an enzyme for cholesterol measurement, such as cholesterol esterase or cholesterol oxidase, is added to liberate and degrade cholesterol in small, dense LDL, and the reaction products is then quantified. In the step of quantification, a surfactant that acts at least on small, dense LDL may be used to quantify cholesterol in small, dense LDL. A surfactant that acts at least on small, dense LDL may be a surfactant that selectively acts on small, dense LDL, a surfactant that acts also on other lipoproteins in addition to small, dense LDL, or a surfactant that acts on all lipoproteins.

As a surfactant that selectively acts on small, dense LDL, a polyoxyethylene-polyoxypropylene copolymer or a derivative thereof can be appropriately used. Examples of the polyoxyethylene-polyoxypropylene copolymer or a derivative thereof include Pluronic^{®}-based surfactants (e.g., BASF and ADEKA Corporation) such as Pluronic 17R-4, Pluronic L-64, Pluronic PE3100, Pluronic P-85, Pluronic F-88, Pluronic P-103, and Pluronic F-127.

An example of a surfactant that acts on all lipoproteins is a polyoxyethylene derivative. Any commercially available surfactant that is used in reagents or the like for total cholesterol measurement can be used. Specific examples include polyoxyethylene alkyl phenyl ether (EMULGEN 909 (Kao Corporation), TritonX-100), polyoxyethylene alkyl ether (EMULGEN 707 (Kao Corporation), and EMULGEN 709 (Kao Corporation)).

The concentration of a surfactant in a reaction solution to be used in the step of quantification of small, dense LDL preferably ranges from approximately 0.01% (w/v) to 10% (w/v) and more preferably ranges from approximately 0.1% (w/v) to 5% (w/v).

Cholesterol esterase activity, cholesterol oxidase activity, sphingomyelinase activity, peroxidase activity, and catalase activity used in the present invention can be measured by the methods described below. Cholesterol oxidase activity is measured using a 6 mM cholesterol solution (dissolved in isopropanol) as a substrate solution. A diluent (0.1 M phosphate buffer, TritonX-100, pH 7.0) is added to adjust the concentration of the analyte to 2 to 4 U/ml, 3 ml of the diluted solution is heated at 37°C for 5 minutes, and 0.05 ml of a substrate solution is then added. Thereafter, the mixture is subjected to the reaction at 37°C, and changes in absorbance at 240 nm are measured. In the present invention, changes in absorbance are measured 2 to 7 minutes after the reaction at 37°C to determine cholesterol oxidase activity. If enzyme activity per unit amount converted from the changes in absorbance measured in the manner described above is 3 U/l or higher, it can be considered that cholesterol oxidase activity is present in the analyte. In other words, the analyte contains "cholesterol oxidase."

Cholesterol esterase activity is measured using a substrate solution (a solution of 0.04% linolenic acid cholesterol, 1% TritonX-100, and 0.6% sodium cholate), a 300 U/ml cholesterol oxidase solution, an enzyme diluent (20 mM phosphate buffer, 0.5 mM EDTA· 2Na, 2 mM MgCl₂, 0.2% BSA, pH 7.5), and a reaction solution (0.06% 4-aminoantipyrine, 0.4% phenol, 7.5 KU/l peroxidase). After 1.75 ml of the reaction solution is mixed with 1.0 ml of the substrate solution, the mixture is heated at 37°C for 5 minutes, and 0.1 ml of a cholesterol oxidase solution is added. After the mixture is heated at 37°C for 2 minutes, 0.1 ml of the analyte diluted with a diluent is added, the mixture is subjected to the reaction at 37°C, and changes in absorbance at 500 nm is then measured. In the present invention, changes in absorbance are measured 0 to 3.5 minutes after the reaction at 37°C to determine cholesterol esterase activity. If enzyme activity per unit amount converted from the changes in absorbance measured in the manner described above is 8 U/l or higher, it can be considered that cholesterol esterase activity is present in the analyte. In other words, the analyte contains "cholesterol esterase."

Sphingomyelinase activity is measured using a reaction solution (a mixture of 0.008% sphingomyeline, 0.05% TritonX-100 solution, 10 U/ml alkaline phosphatase, 10 U/ml cholesterol oxidase, 2 U/ml peroxidase, 0.02% 4-aminoantipyrine, and 0.02% TODB), a reaction terminator (a 1% sodium dodecyl sulfate solution), and a diluent (10 mM Tris buffer, 0.1% TritonX-100, pH 8.0). The reaction solution (0.08 ml) is mixed with 0.003 ml of the analyte diluted with a diluent, the mixture is heated at 37°C for 5 minutes, and 0.16 ml of the reaction terminator is then added. After the reaction is terminated, changes in absorbance are measured at a dominant wavelength of 546 nm and a subwavelength of 700 nm to determine sphingomyelinase activity. If enzyme activity per unit amount converted from the changes in absorbance measured in the manner described above is 2 U/l or higher, it can be considered that sphingomyelinase activity is present in the analyte. In other words, the analyte contains "sphingomyelinase."

Peroxidase activity is measured using a reaction solution 1 (1.5 mM HDAOS, 0.05% TritonX-100, 50 mM phosphate buffer, pH 7.0), a reaction solution 2 (5 mM 4-aminoantipyrine, 0.05% TritonX-100, 1% hydrogen peroxide, 50 mM phosphate buffer, pH 7.0), and a diluent (50 mM phosphate buffer, pH 7.0). The reaction solution 1 (0.3 ml) is mixed with 0.08 ml of the analyte diluted with a diluent, and the mixture is heated at 37°C for 5 minutes. Thereafter, 0.1 ml of the reaction solution 2 is added, the reaction is allowed to proceed at 37°C, and changes in absorbance are measured at a dominant wavelength of 600 nm and a subwavelength of 700 nm. In the present invention, changes in absorbance are measured 2 to 5 minutes after the reaction at 37°C to determine peroxidase activity. If enzyme activity per unit amount converted from the changes in absorbance measured in the manner described above is 10 U/l or higher, it can be considered that peroxidase activity is present in the analyte. In other words, the analyte contains "peroxidase."

Catalase activity is measured using a substrate solution (0.06% hydrogen peroxide, 50 mM phosphate buffer, pH 7.0). After 2.9 ml of the substrate solution is pre-heated at 25°C, the solution is mixed with 0.1 ml of the analyte, and changes in absorbance at 240 nm are measured. In the present invention, changes in absorbance are measured 0 to 3 minutes after the reaction at 25°C to determine catalase activity. If enzyme activity per unit amount converted from the changes in absorbance measured in the manner described above is 100 U/l or higher, it can be considered that catalase activity is present in the analyte. In other words, the analyte contains "catalase."

In the step of eliminating cholesterol in lipoproteins other than small, dense LDL and leading such cholesterol to the outside of the reaction system according to the present invention, a monovalent cation and/or a divalent cation or a salt thereof can be used as an ionic strength adjuster. Addition of such ionic strength adjuster facilitates differentiation of small, dense LDL from L LDL. Specifically, sodium chloride, potassium chloride, magnesium chloride, manganese chloride, calcium chloride, lithium chloride, ammonium chloride, magnesium sulfate, potassium sulfate, lithium sulfate, ammonium sulfate, magnesium acetate, and the like can be used. The concentration of such ionic strength adjuster at the time of the reaction preferably ranges from 0 mmol/l to 100 mmol/.

In the present invention, moreover, polyanion can be added in the step of eliminating cholesterol in L LDL and leading such cholesterol to the outside of the reaction system and the step of eliminating cholesterol in lipoproteins other than LDL and leading such cholesterol to the outside of the reaction system in order to adjust the catalytic activity of sphingomyelinase against small, dense LDL and L LDL. As polyanion to be added, heparin, phosphotungstic acid, dextran sulfate, or the like can be preferably used. In the case of heparin, the concentration of polyanion in a reagent preferably ranges from 10 U/ml to 250 U/ml. In the case of phosphotungstic acid, it preferably ranges from 0.02% (w/v) to 1.25% (w/v). In the case of dextran sulfate, it preferably ranges from 0.02% (w/v) to 1.25% (w/v). The concentrations of polyanions in reaction solutions preferably range from 5 U/ml to 250 U/ml, 0.01% (w/v) to 1.25% (w/v), and 0.01% (w/v) to 1.25% (w/v), respectively.

The enzyme in the reagent can also be identified in the manner described below. Specifically, a sample containing a target enzyme is degraded with trypsin, and the resulting fragment peptide is detected using a hybrid mass spectrometer. The peptide mass determined using a mass spectrometer and the spectra of fragment ions obtained in collision with argon gas within a mass spectrometer (MS/MS data) are applied to database search (e.g., Mascot search). Thus, a protein can be identified. When a sequence of a fragment peptide derived from an amino acid sequence in the sample is consistent with the amino acid sequence registered with the database as an unique sequence, the sample can be considered to contain a target enzyme.

The enzyme in the reagent can also be identified in the manner described below. From among fragment peptides obtained by degrading a target enzyme with trypsin, specifically, a peptide that is specific to a target enzyme and exhibits an intense signal detected by mass spectrometry is selected as a target peptide to be quantified. Concerning the target peptide to be quantified, a non-labeled peptide and a peptide labeled with a stable isotope as the internal standard are prepared via chemical synthesis. A sample containing a target enzyme is completely digested with trypsin, a known amount of a stable isotope-labeled peptide is added, and the sample is analyzed using a triple quadrupole mass spectrometer (LC-MS/MS) connected to HPLC in the MRM mode (i.e., the multiple reaction monitoring mode). A mixture containing a non-labeled target peptide and a known amount of a stable isotope-labeled peptide is analyzed in the same manner, a calibration curve indicating the concentration ratio and the peak area ratio relative to the internal standard is prepared, and the absolute amount of the target peptide in the sample is determined. Thus, the target peptide can be quantified.

The reaction in each step of the present invention is preferably carried out at a temperature between 2°C and 45°C and more preferably between 25°C and 40°C.

The reaction in each step is preferably carried out for 1 to 10 minutes and more preferably for 3 to 7 minutes.

Serum and blood plasma samples can be used as samples obtained from a subject in the present invention, although samples are not limited thereto.

Examples of an autoanalyzer to be used in the present invention include TBA-120FR·200FR (TOSHIBA Corporation), JCA-BM 1250·1650·2250 (JEOL Ltd.), HITACHI 7180·7170 (Hitachi, Ltd.), AU 2700·5800·680 (OLYMPUS), and cobas c501·701 (Roche).

When there are 2 steps; i.e., a step of eliminating cholesterol in lipoproteins other than small, dense LDL and leading such cholesterol to the outside of the reaction system and a step of measuring small, dense LDL, a reagent used in the step of eliminating cholesterol in lipoproteins other than small, dense LDL and leading such cholesterol to the outside of the reaction system contains sphingomyelinase that reacts with lipoproteins other than small, dense LDL. A reagent composition for eliminating cholesterol in lipoproteins other than small, dense LDL may further be supplemented with an enzyme that degrades cholesterol, such as cholesterol esterase or cholesterol oxidase, either of an electron donor or a coupler, catalase for eliminating hydrogen peroxide, and the like. A reagent used in the step of measuring small, dense LDL may be supplemented with a surfactant that selectively reacts with small, dense LDL or a surfactant that reacts with all lipoproteins and either of an electron donor or a coupler, which is not used in the step of eliminating cholesterol in lipoproteins other than small, dense LDL and leading such cholesterol to the outside of the reaction system. In such a case, a monovalent cation, a divalent cation, a salt of the monovalent or divalent cation, or a polyanion may also be added to the first reagent and the second reagent, according to need. Also, the first reagent and the second reagent may contain serum albumin. The pH of each reagent composition may be around neutral pH and ranges, for example, from pH 6 to pH 8 and preferably ranges from pH 6.5 to pH 7.5. The pH may be adjusted with the addition of a buffer.

When the method of the present invention comprises performing the step of eliminating cholesterol in lipoproteins other than small, dense LDL and leading such cholesterol to the outside of the reaction system and the step of measuring small, dense LDL in that order, a reagent composition for eliminating cholesterol in lipoproteins other than small, dense LDL may be first added to a sample obtained from a subject for reaction, a reagent composition for measuring small, dense LDL may be added for further reaction, and the absorbance may then be measured.

Although the amount of a sample and the amount of each reagent composition are not particularly limited, and can be adequately determined in view of the concentration or the like of a reagent in each reagent composition, they are determined within the amounts which can be applied to an autoanalyzer. For example, 1 µl to 10 µl of a sample obtained from a subject, 50 to 300 µl of the first reagent, and 25 to 200 µl of the second reagent may be used.

### Examples

Hereafter, the present invention is described in greater detail with reference to the examples, although the present invention is not limited to the examples described below.

Ingredients used in Examples and Comparative Examples are as described below.

In Examples and Comparative Examples below, absorption spectra were measured using HITACHI U-3900.

### Comparative Example 1

A second reagent composition used in the second step of the method for measuring small, dense LDL comprising the first step of eliminating cholesterol in lipoproteins other than small, dense LDL and the second step of measuring small, dense LDL cholesterol was prepared as described below.

### Second reagent composition

PIPES buffer (pH 7.0): 50 mmol/l
Coupler (4-aminoantipyrine): 4.0 mmol/l
Peroxidase: 5.0U/ml
Potassium ferrocyanide: 0.11 mmol/l
Sodium azide: 0.05% (w/v)
Polyoxyethylene alkyl ether (Kao Corporation): 1.0% (w/v)

The second reagent composition was subjected to accelerated storage at 37°C for 1 week or 2 weeks, and spontaneous color development during storage was inspected by measuring the absorption spectra at the wavelengths ranging from 320 nm to 480 nm.

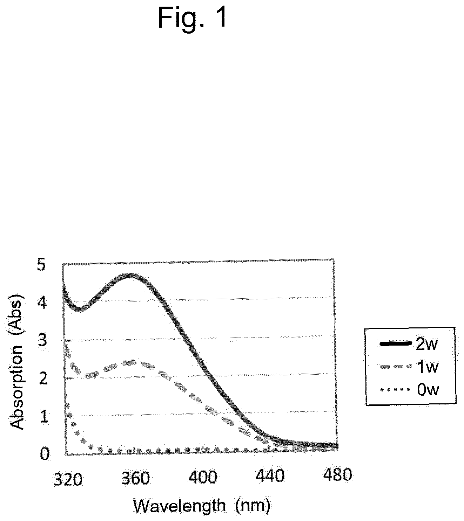

Fig. 1 shows the absorption spectra immediately after preparation and after accelerated storage at 37°C for 1 week or 2 weeks. As shown in Fig. 1, the reagent after accelerated storage at 37°C for 1 week or 2 weeks exhibited a peak indicating the maximum absorption at around 360 nm (the reagent became pale yellow). The results of accelerated storage at 37°C for 1 week were equivalent to the results of refrigeration for 1.5 years.

### Comparative Example 2

A first reagent composition used in the first step of the method for measuring small, dense LDL comprising the first step of eliminating cholesterol in lipoproteins other than small, dense LDL and the second step of measuring small, dense LDL cholesterol was prepared as described below.

### First reagent composition

PIPES buffer (pH 7.0): 50 mmol/l
Sphingomyelinase: 2.7 U/ml
Cholesterol esterase: 0.6 U/ml
Cholesterol oxidase: 0.5 U/ml
Coupler (4-aminoantipyrine): 1.3 mmol/l
Peroxidase: 1.7 U/ml
Potassium ferrocyanide: 0.04 mmol/l
Polyoxyethylene derivative (Kao Corporation): 0.3% (w/v)

The first reagent composition was subjected to accelerated storage at 37°C for 1 week or 2 weeks, and spontaneous color development during storage was inspected by measuring the absorption spectra at the wavelengths ranging from 320 nm to 480 nm.

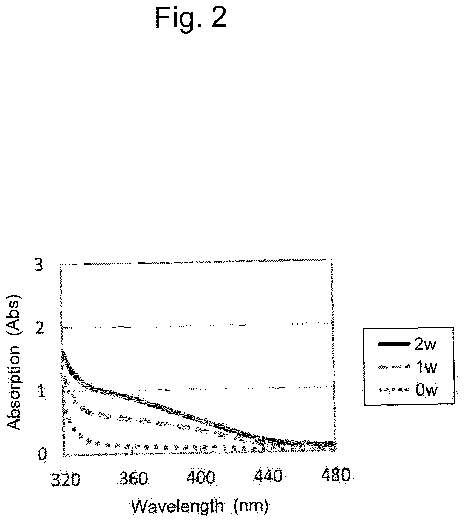

Fig. 2 shows the absorption spectra immediately after preparation and after accelerated storage at 37°C for 1 week or 2 weeks. As shown in Fig. 2, the reagent after accelerated storage at 37°C exhibited a peak indicating the maximum absorption at around 360 nm (the reagent became pale yellow).

### Example 1

A first reagent composition used in the first step and a second reagent composition used in the second step of the method for measuring small, dense LDL comprising the first step of eliminating cholesterol in lipoproteins other than small, dense LDL and the second step of measuring small, dense LDL cholesterol were prepared as described below.

### First reagent composition

PIPES buffer (pH 7.0): 50 mmol/l
Sphingomyelinase: 2.7 U/ml
Cholesterol esterase: 0.6 U/ml
Cholesterol oxidase: 0.5 U/ml
Catalase: 1200 U/ml
4-Aminoantipyrine: 1.3 mmol/l
Polyoxyethylene polycyclic phenyl ether (Kao Corporation): 0.3% (w/v)
Second reagent composition
PIPES buffer (pH 7.0): 50 mmol/l
TOOS: 6.0 mmol/l
Potassium ferrocyanide: 0.11 mM
Peroxidase: 5.0 U/ml
Sodium azide: 0.05% (w/v)
Polyoxyethylene alkyl ether (Kao Corporation): 1.0% (w/v)

The first reagent composition and the second reagent composition were subjected to accelerated storage at 37°C for 1 week or 2 weeks, spontaneous color development during storage was inspected by measuring the absorption spectra at the wavelengths ranging from 320 nm to 480 nm, and the results were compared with the results of Comparative Examples 1 and 2.

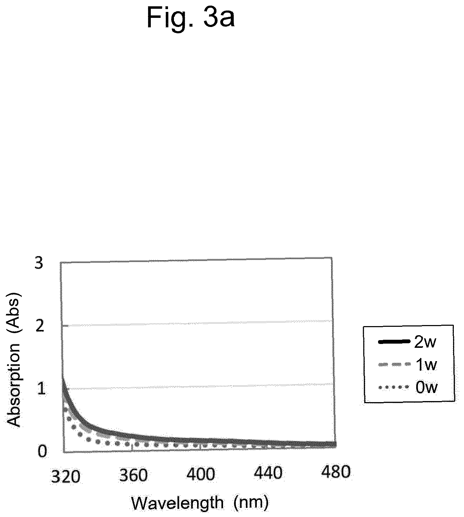

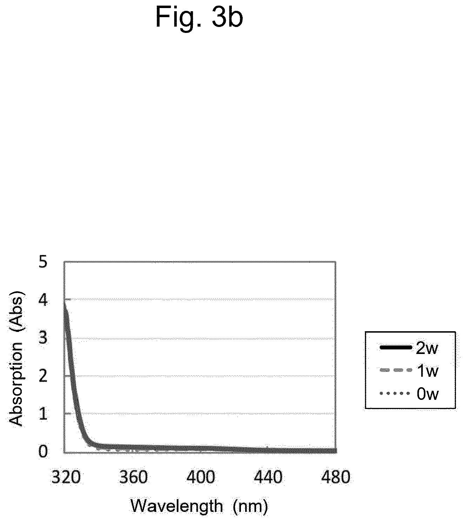

Fig. 3a shows the absorption spectra of the first reagent and Fig. 3b shows the absorption spectra of the second reagent immediately after preparation and after accelerated storage at 37°C for 1 week or 2 weeks. As shown in Fig. 3a and Fig. 3b, peaks indicating the maximum absorption at around 360 nm were reduced or disappeared, in comparison with Comparative Examples 1 and 2.

### Example 2

A first reagent composition used in the first step and a second reagent composition used in the second step of the method for measuring small, dense LDL comprising the first step of eliminating cholesterol in lipoproteins other than small, dense LDL and the second step of measuring small, dense LDL cholesterol were prepared as described below.

### First reagent composition

PIPES buffer (pH 7.0): 50 mmol/l
Sphingomyelinase: 2.7 U/ml
Cholesterol esterase: 0.6 U/ml
Cholesterol oxidase: 0.5 U/ml
Peroxidase: 1.7 U/ml
4-Aminoantipyrine: 1.3 mmol/l
Polyoxyethylene polycyclic phenyl ether (Kao Corporation): 0.3% (w/v)
Second reagent composition
PIPES buffer (pH 7.0): 50 mmol/l
TOOS: 6.0 mmol/l
Polyoxyethylene alkyl ether (Kao Corporation): 1.0% (w/v)
Potassium ferrocyanide: 0.11mmol/l

The first reagent composition and the second reagent composition were subjected to accelerated storage at 37°C for 1 week or 2 weeks, spontaneous color development during storage was inspected by measuring the absorption spectra at the wavelengths ranging from 320 nm to 480 nm, and the results were compared with the results of Comparative Examples 1 and 2.

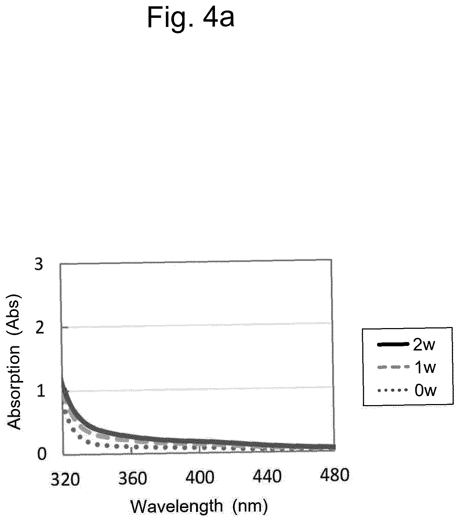

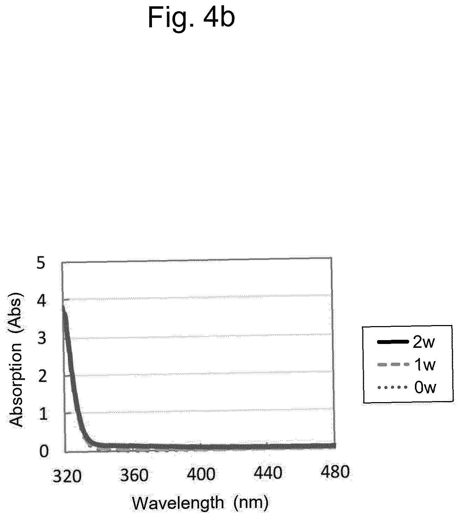

Fig. 4a shows the absorption spectra of the first reagent and Fig. 4b shows the absorption spectra of the second reagent immediately after preparation and after accelerated storage at 37°C for 1 week or 2 weeks. As shown in Fig. 4a and Fig. 4b, peaks indicating the maximum absorption at around 360 nm were reduced or disappeared, in comparison with Comparative Examples 1 and 2.

### Example 3

A first reagent composition used in the first step and a second reagent composition used in the second step of the method for measuring small, dense LDL comprising the first step of eliminating cholesterol in lipoproteins other than small, dense LDL and the second step of measuring small, dense LDL cholesterol were prepared as described below.

### First reagent composition

PIPES buffer (pH 7.0): 50 mmol/l
Sphingomyelinase: 2.7 U/ml
Cholesterol esterase: 0.6 U/ml
Cholesterol oxidase: 0.5 U/ml
4-Aminoantipyrine: 1.3 mmol/l
Potassium ferrocyanide: 0.04 mM
Catalase: 1200 U/ml
Polyoxyethylene polycyclic phenyl ether (Kao Corporation): 0.3% (w/v)
Second reagent composition
PIPES buffer (pH 7.0): 50 mmol/l
TOOS: 6.0 mmol/l
Sodium azide: 0.05% (w/v)
Peroxidase: 5.0U/ml
Polyoxyethylene alkyl ether (Kao Corporation): 1.0% (w/v)

The first reagent composition and the second reagent composition were subjected to accelerated storage at 37°C for 1 week or 2 weeks, spontaneous color development during storage was inspected by measuring the absorption spectra at the wavelengths ranging from 320 nm to 480 nm, and the results were compared with the results of Comparative Examples 1 and 2.

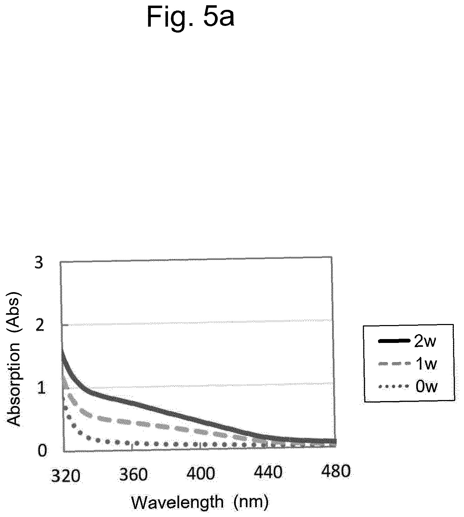

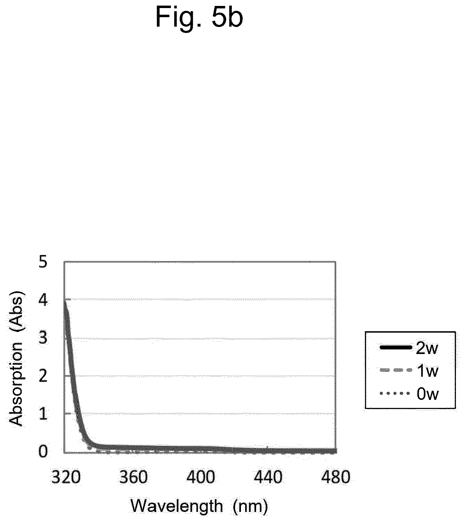

Fig. 5a shows the absorption spectra of the first reagent and Fig. 5b shows the absorption spectra of the second reagent immediately after preparation and after accelerated storage at 37°C for 1 week or 2 weeks. As shown in Fig. 5a and Fig. 5b, peaks indicating the maximum absorption at around 360 nm were reduced or disappeared, in comparison with Comparative Examples 1 and 2.

### Example 4

A first reagent composition used in the first step and a second reagent composition used in the second step of the method for measuring small, dense LDL comprising the first step of eliminating cholesterol in lipoproteins other than small, dense LDL and the second step of measuring small, dense LDL cholesterol were prepared as described below.

### First reagent composition

PIPES buffer (pH 7.0): 50 mmol/l
Sphingomyelinase: 2.7 U/ml
Cholesterol esterase: 0.6 U/ml
Cholesterol oxidase: 0.5 U/ml
Peroxidase: 1.7 U/ml
TOOS: 2.0 mmol/l
Polyoxyethylene polycyclic phenyl ether (Kao Corporation): 0.3% (w/v)
Second reagent composition
PIPES buffer (pH 7.0): 50 mmol/l
4-Aminoantipyrine: 4.0 mmol/l
Potassium ferrocyanide: 0.11 mM
Polyoxyethylene alkyl ether (Kao Corporation): 1.0% (w/v)

The first reagent composition and the second reagent composition were subjected to accelerated storage at 37°C for 1 week or 2 weeks, spontaneous color development during storage was inspected by measuring the absorption spectra at the wavelengths ranging from 320 nm to 480 nm, and the results were compared with the results of Comparative Examples 1 and 2.

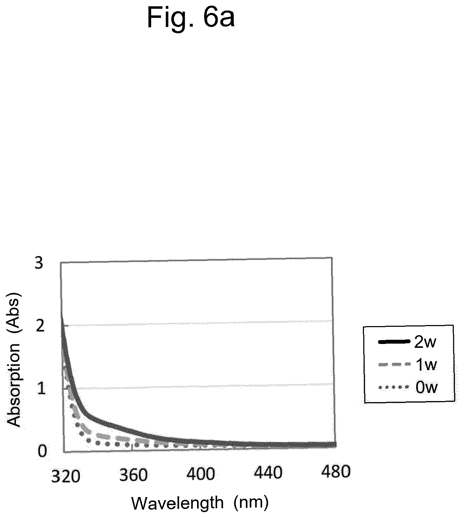

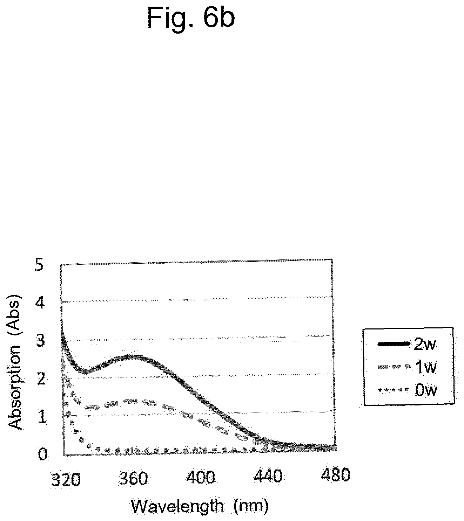

Fig. 6a shows the absorption spectra of the first reagent and Fig. 6b shows the absorption spectra of the second reagent immediately after preparation and after accelerated storage at 37°C for 1 week or 2 weeks. As shown in Fig. 6a and Fig. 6b, peaks indicating the maximum absorption at around 360 nm were reduced or disappeared, in comparison with Comparative Examples 1 and 2.

### Example 5

A first reagent composition used in the first step and a second reagent composition used in the second step of the method for measuring small, dense LDL comprising the first step of eliminating cholesterol in lipoproteins other than small, dense LDL and the second step of measuring small, dense LDL cholesterol were prepared as described below.

### First reagent composition

PIPES buffer (pH 7.0): 50 mmol/l
Sphingomyelinase: 2.7 U/ml
Cholesterol esterase: 0.6 U/ml
Cholesterol oxidase: 0.5 U/ml
TOOS: 2.0 mmol/l
Potassium ferrocyanide: 0.04 mM
Catalase: 1200 U/ml
Polyoxyethylene polycyclic phenyl ether (Kao Corporation): 0.3% (w/v)
Second reagent composition
PIPES buffer (pH 7.0): 50 mmol/l
4-Aminoantipyrine: 4.0 mmol/l
Peroxidase: 5.0 U/ml
Sodium azide: 0.05% (w/v)
Polyoxyethylene alkyl ether (Kao Corporation): 1.0% (w/v)

The first reagent composition and the second reagent composition were subjected to accelerated storage at 37°C for 1 week or 2 weeks, spontaneous color development during storage was inspected by measuring the absorption spectra at the wavelengths ranging from 320 nm to 480 nm, and the results were compared with the results of Comparative Examples 1 and 2.

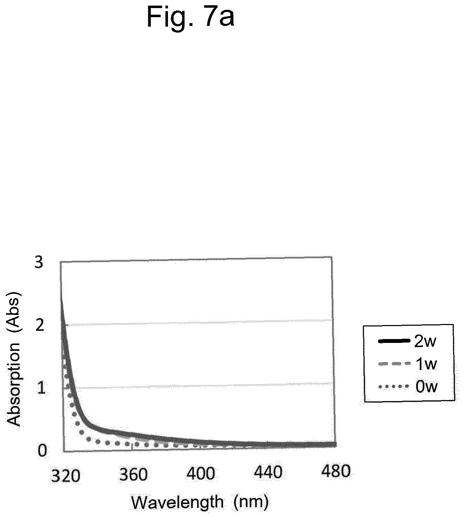

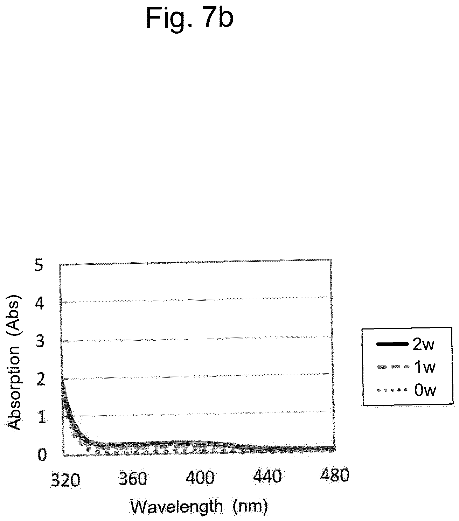

Fig. 7a shows the absorption spectra of the first reagent and Fig. 7b shows the absorption spectra of the second reagent immediately after preparation and after accelerated storage at 37°C for 1 week or 2 weeks. As shown in Fig. 7a and Fig. 7b, peaks indicating the maximum absorption at around 360 nm were reduced or disappeared, in comparison with Comparative Examples 1 and 2.

### Example 6

A first reagent composition used in the first step and a second reagent composition used in the second step of the method for measuring small, dense LDL comprising the first step of eliminating cholesterol in lipoproteins other than small, dense LDL and the second step of measuring small, dense LDL cholesterol were prepared as described below.

### First reagent composition

PIPES buffer (pH 7.0): 50 mmol/l
Sphingomyelinase: 2.7 U/ml
Cholesterol esterase: 0.6 U/ml
Cholesterol oxidase: 0.5 U/ml
TOOS: 2.0 mmol/l
Potassium ferrocyanide: 0.04 mM
Peroxidase: 1.7 U/ml
Polyoxyethylene polycyclic phenyl ether (Kao Corporation): 0.3% (w/v)
Second reagent composition
PIPES buffer (pH 7.0): 50 mmol/l
4-Aminoantipyrine: 4.0 mmol/l
Polyoxyethylene alkyl ether (Kao Corporation): 1.0% (w/v)

The first reagent composition and the second reagent composition were subjected to accelerated storage at 37°C for 1 week or 2 weeks, spontaneous color development during storage was inspected by measuring the absorption spectra at the wavelengths ranging from 320 nm to 480 nm, and the results were compared with the results of Comparative Examples 1 and 2.

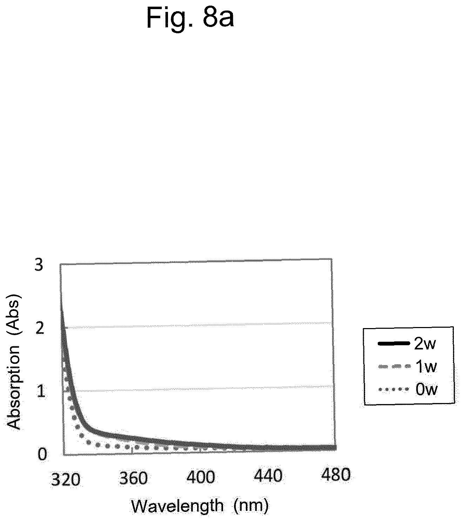

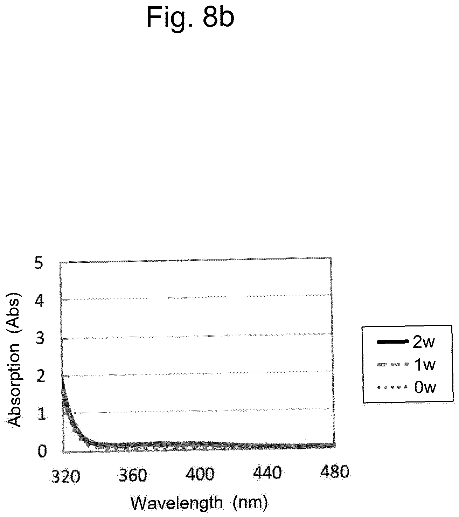

Fig. 8a shows the absorption spectra of the first reagent and Fig. 8b shows the absorption spectra of the second reagent immediately after preparation and after accelerated storage at 37°C for 1 week or 2 weeks. As shown in Fig. 8a and Fig. 8b, peaks indicating the maximum absorption at around 360 nm were reduced or disappeared, in comparison with Comparative Examples 1 and 2.

### Example 7

A first reagent composition used in the first step and a second reagent composition used in the second step of the method for measuring small, dense LDL comprising the first step of eliminating cholesterol in lipoproteins other than small, dense LDL and the second step of measuring small, dense LDL cholesterol were prepared as described below.

### First reagent composition

PIPES buffer (pH 7.0): 50 mmol/l
Sphingomyelinase: 2.7 U/ml
Cholesterol esterase: 0.6 U/ml
Cholesterol oxidase: 0.5 U/ml
4-Aminoantipyrine: 1.3 mmol/l
Peroxidase: 1.7 U/ml
Catalase: 1200 U/ml
Polyoxyethylene polycyclic phenyl ether (Kao Corporation): 0.3% (w/v)
Second reagent composition
PIPES buffer (pH 7.0): 50 mmol/l
TOOS: 6.0 mmol/l
Potassium ferrocyanide: 0.11 mM
Sodium azide: 0.05% (w/v)
Polyoxyethylene alkyl ether (Kao Corporation): 1.0% (w/v)

The first reagent composition and the second reagent composition were subjected to accelerated storage at 37°C for 1 week or 2 weeks, spontaneous color development during storage was inspected by measuring the absorption spectra at the wavelengths ranging from 320 nm to 480 nm, and the results were compared with the results of Comparative Examples 1 and 2.

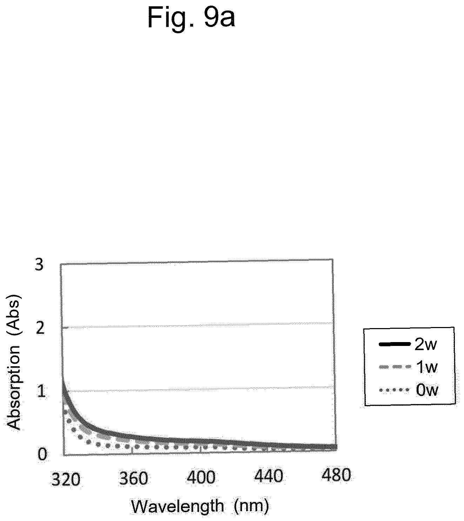

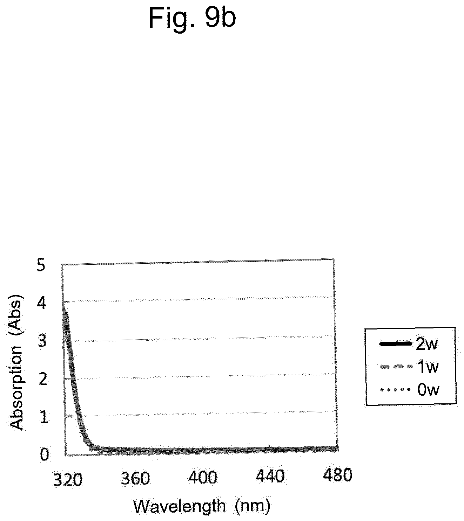

Fig. 9a shows the absorption spectra of the first reagent and Fig. 9b shows the absorption spectra of the second reagent immediately after preparation and after accelerated storage at 37°C for 1 week or 2 weeks. As shown in Fig. 9a and Fig. 9b, peaks indicating the maximum absorption at around 360 nm were reduced or disappeared, in comparison with Comparative Examples 1 and 2.

### Example 8

A first reagent composition used in the first step and a second reagent composition used in the second step of the method for measuring small, dense LDL comprising the first step of eliminating cholesterol in lipoproteins other than small, dense LDL and the second step of measuring small, dense LDL cholesterol were prepared as described below.

### First reagent composition

PIPES buffer (pH 7.0): 50 mmol/l
Sphingomyelinase: 2.7 U/ml
Cholesterol esterase: 0.6 U/ml
Cholesterol oxidase: 0.5 U/ml
TOOS: 2.0 mmol/l
Peroxidase: 1.7 U/ml
Catalase: 1200 U/ml
Polyoxyethylene polycyclic phenyl ether (Kao Corporation): 0.3% (w/v)
Second reagent composition
PIPES buffer (pH 7.0): 50 mmol/l
4-Aminoantipyrine: 4.0 mmol/l
Potassium ferrocyanide: 0.11 mM
Sodium azide: 0.05% (w/v)
Polyoxyethylene alkyl ether (Kao Corporation): 1.0% (w/v)

The first reagent composition and the second reagent composition were subjected to accelerated storage at 37°C for 1 week or 2 weeks, spontaneous color development during storage was inspected by measuring the absorption spectra at the wavelengths ranging from 320 nm to 480 nm, and the results were compared with the results of Comparative Examples 1 and 2.

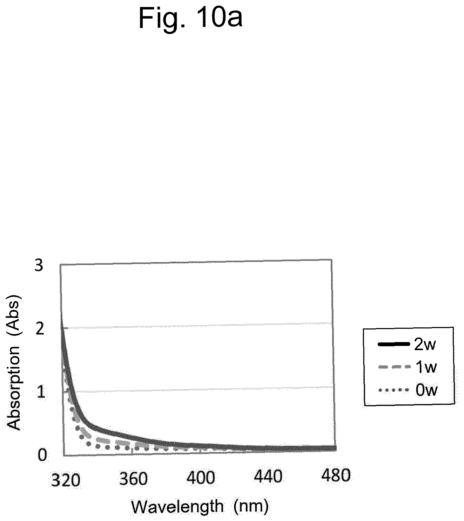

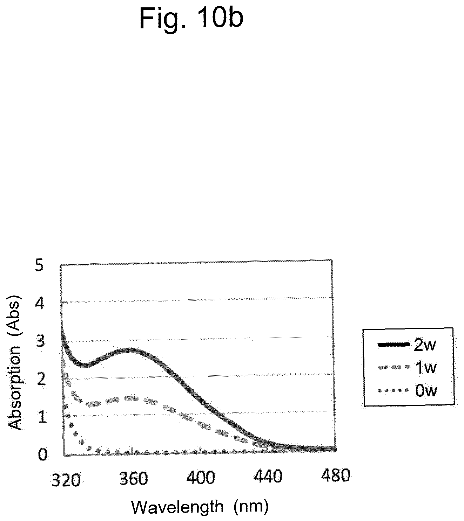

Fig. 10a shows the absorption spectra of the first reagent and Fig. 10b shows the absorption spectra of the second reagent immediately after preparation and after accelerated storage at 37°C for 1 week or 2 weeks. As shown in Fig. 10a and Fig. 10b, peaks indicating the maximum absorption at around 360 nm were reduced or disappeared, in comparison with Comparative Examples 1 and 2.

### Example 9

A first reagent composition used in the first step and a second reagent composition used in the second step of the method for measuring small, dense LDL comprising the first step of eliminating cholesterol in lipoproteins other than small, dense LDL and the second step of measuring small, dense LDL cholesterol were prepared as described below.

### First reagent composition

PIPES buffer (pH 7.0): 50 mmol/l
Sphingomyelinase: 2.7 U/ml
Cholesterol esterase: 0.6 U/ml
Cholesterol oxidase: 0.5 U/ml
TOOS: 2.0 mmol/l
Potassium ferrocyanide: 0.04 mM
Peroxidase: 1.7 U/ml
Catalase: 1200 U/ml
Polyoxyethylene polycyclic phenyl ether (Kao Corporation): 0.3% (w/v)
Second reagent composition
PIPES buffer (pH 7.0): 50 mmol/l
4-Aminoantipyrine: 4.0 mmol/l
Sodium azide: 0.05% (w/v)
Polyoxyethylene alkyl ether (Kao Corporation): 1.0% (w/v)

The first reagent composition and the second reagent composition were subjected to accelerated storage at 37°C for 1 week or 2 weeks, spontaneous color development during storage was inspected by measuring the absorption spectra at the wavelengths ranging from 320 nm to 480 nm, and the results were compared with the results of Comparative Examples 1 and 2.

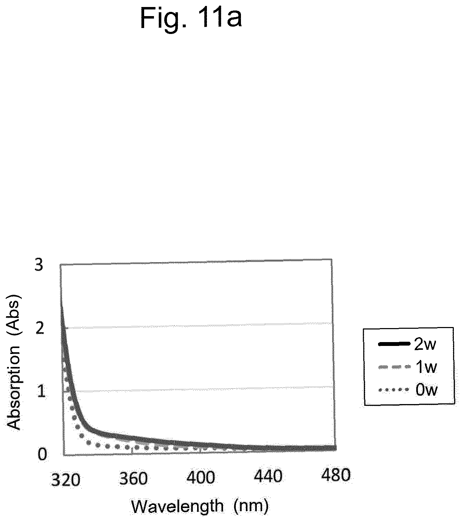

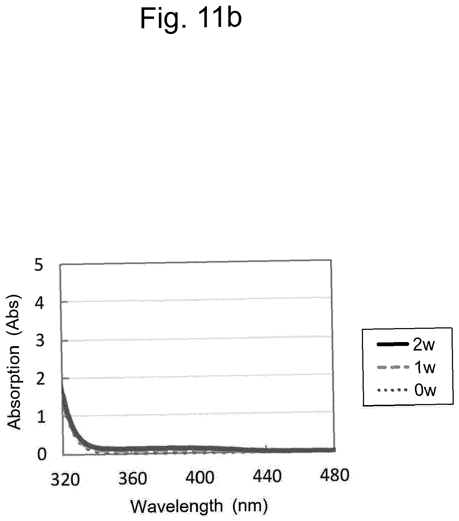

Fig. 11a shows the absorption spectra of the first reagent and Fig. 11b shows the absorption spectra of the second reagent immediately after preparation and after accelerated storage at 37°C for 1 week or 2 weeks. As shown in Fig. 11a and Fig. 11b, peaks indicating the maximum absorption at around 360 nm were reduced or disappeared, in comparison with Comparative Examples 1 and 2.

### Examination 1

With the use of the kits for quantification of Example 1 to Example 9, small, dense LDL cholesterol was measured. For comparison, the reagent measuring the level of small, dense LDL cholesterol, the sd LDL-EX "SEIKEN" (Denka Seiken Co., Ltd.), was used. The levels of small, dense LDL cholesterol were compared. Table 1 shows the correlation coefficients between the results of measurement of cholesterol in small, dense LDL of the same sample obtained with the use of the reagents prepared in the examples and the results of measurement obtained with the use of the sd LDL-EX "SEIKEN."

As shown in Table 1, a good correlation was observed between the methods of the examples of the present invention and the method involving the use of the reagent measuring the level of small, dense LDL cholesterol, the sd LDL-EX "SEIKEN."

**[Table 1]**

| Example | Correlation coefficient (r) |
|---|---|
| Example 1 | 0.999 |
| Example 2 | 0.997 |
| Example 3 | 0.999 |
| Example 4 | 0.996 |
| Example 5 | 1.000 |
| Example 6 | 0.992 |
| Example 7 | 0.996 |
| Example 8 | 0.997 |
| Example 9 | 0.983 |

The results of the examination described above demonstrate that the kit for quantification of the present invention enables satisfactory detection of cholesterol in sd LDL.

### Industrial Applicability

With the use of the kit and the method of the present invention, cholesterol in small, dense LDL can be quantified without damaging reagent stability.

All publications, patents, and patent applications cited herein are incorporated herein by reference in their entirety.

## Claims

1. A kit for quantification of cholesterol in small, dense LDL in a sample obtained from a subject used in a method for quantifying cholesterol in small, dense LDL in two steps comprising:
(1) a first reagent composition having cholesterol esterase activity, cholesterol oxidase activity, and sphingomyelinase activity and leading cholesterol in lipoproteins other than small, dense LDL to the outside of the reaction system in the presence of cholesterol esterase activity, cholesterol oxidase activity, and sphingomyelinase activity; and
(2) a second reagent composition for quantifying cholesterol in small, dense LDL,
wherein a coupler, an iron complex, and peroxidase are not allowed to be present in the same reagent composition, which is either the first reagent composition or the second reagent composition.

2. A kit for quantification of small, dense LDL cholesterol in a sample obtained from a subject used in a method for quantifying cholesterol in small, dense LDL in two steps comprising:
(1) a first reagent composition having cholesterol esterase activity, cholesterol oxidase activity, and sphingomyelinase activity and leading cholesterol in lipoproteins other than small, dense LDL to the outside of the reaction system in the presence of cholesterol esterase activity, cholesterol oxidase activity, and sphingomyelinase activity; and
(2) a second reagent composition for quantifying cholesterol in small, dense LDL,
wherein a coupler and an iron complex are not allowed to be present in the same reagent composition, which is either the first reagent composition or the second reagent composition.

3. The kit according to Claim 1 or 2, wherein the first reagent composition comprises catalase activity and a coupler and the second reagent composition comprises an electron donor, an iron complex, and peroxidase activity.

4. The kit according to Claim 1 or 2, wherein the first reagent composition comprises peroxidase activity and a coupler and the second reagent composition comprises an electron donor and an iron complex.

5. The kit according to Claim 1, wherein the first reagent composition comprises catalase activity, a coupler, and an iron complex and the second reagent composition comprises an electron donor and peroxidase activity.

6. The kit according to Claim 1, wherein the first reagent composition comprises peroxidase activity and an electron donor and the second reagent composition comprises a coupler and an iron complex.

7. The kit according to Claim 1 or 2, wherein the first reagent composition comprises catalase activity, an electron donor, and an iron complex and the second reagent composition comprises peroxidase activity and a coupler.

8. The kit according to Claim 1 or 2, wherein the first reagent composition comprises peroxidase activity, an electron donor, and an iron complex and the second reagent composition comprises a coupler.

9. The kit according to any one of Claims 4, 6, and 8, wherein the first reagent composition further comprises catalase activity.

10. The kit according to any one of Claims 1 to 9, wherein the first reagent composition further comprises a surfactant that acts on lipoproteins other than small, dense LDL and the second reagent composition further comprises a surfactant that acts at least on small, dense LDL.

11. The kit according to Claim 10, wherein the surfactant contained in the first reagent composition comprises polyoxyethylene polycyclic phenyl ether.

12. The kit according to Claim 11, wherein the polyoxyethylene polycyclic phenyl ether includes a polyoxyethylene benzyl phenyl ether derivative and/or a polyoxyethylene styrenated phenyl ether derivative.

13. A method for quantifying cholesterol in small, dense LDL in a sample obtained from a subject in two steps comprising:
(1) a first step of leading cholesterol in lipoproteins other than small, dense LDL to the outside of the reaction system in the presence of cholesterol esterase activity, cholesterol oxidase activity, and sphingomyelinase activity; and
(2) a second step of quantifying cholesterol in lipoproteins remaining after the first step,
wherein, in either the step (1) or (2), the coupler, the iron complex, and peroxidase are not used simultaneously.

14. A method for quantifying cholesterol in small, dense LDL in a sample obtained from a subject in two steps comprising:
(1) a first step of leading cholesterol in lipoproteins other than small, dense LDL to the outside of the reaction system in the presence of cholesterol esterase activity, cholesterol oxidase activity, and sphingomyelinase activity; and
(2) a second step of quantifying cholesterol in lipoproteins remaining after the first step,
wherein, in either the step (1) or (2), the coupler and the iron complex are not used simultaneously.

15. The method of quantification according to Claim 13 or 14, wherein the first step involves the use of catalase activity and a coupler and the second step involves the use of an electron donor, an iron complex, and peroxidase activity.

16. The method of quantification according to Claim 13 or 14, wherein the first step involves the use of peroxidase activity and a coupler and the second step involves the use of an electron donor and an iron complex.

17. The method of quantification according to Claim 13, wherein the first step involves the use of catalase activity, a coupler, and an iron complex and the second step involves the use of an electron donor and peroxidase activity.

18. The method of quantification according to Claim 13, wherein the first step involves the use of peroxidase activity and an electron donor and the second step involves the use of a coupler and an iron complex.

19. The method of quantification according to Claim 13 or 14, wherein the first step involves the use of catalase activity, an electron donor, and an iron complex and the second step involves the use of peroxidase activity and a coupler.

20. The method of quantification according to Claim 13 or 14, wherein the first step involves the use of peroxidase activity, an electron donor, and an iron complex and the second step involves the use of a coupler.

21. The method of quantification according to any one of Claims 14, 18, and 20, wherein the first step further involves the use of catalase activity.

22. The method of quantification according to any one of Claims 13 to 21, wherein the first step further involves the use of a surfactant that acts on lipoproteins other than small, dense LDL and the second step further involves the use of a surfactant that acts at least on small, dense LDL.

23. Use of a first reagent composition and a second reagent composition for producing a kit for quantification of small, dense LDL cholesterol in a sample obtained from a subject in the method for quantifying cholesterol in small, dense LDL in two steps:
(1) a first reagent composition having cholesterol esterase activity, cholesterol oxidase activity, and sphingomyelinase activity and leading cholesterol in lipoproteins other than small, dense LDL to the outside of the reaction system in the presence of cholesterol esterase activity, cholesterol oxidase activity, and sphingomyelinase activity; and
(2) a second reagent composition for quantifying cholesterol in small, dense LDL,
wherein a coupler, an iron complex, and peroxidase are not allowed to be present in the same reagent composition, which is either the first reagent composition or the second reagent composition.

24. Use of a first reagent composition and a second reagent composition for producing a kit for quantification of small, dense LDL cholesterol in a sample obtained from a subject in the method for quantifying cholesterol in small, dense LDL in two steps:
(1) a first reagent composition having cholesterol esterase activity, cholesterol oxidase activity, and sphingomyelinase activity; and
(2) a second reagent composition for quantifying cholesterol in small, dense LDL,
wherein a coupler and an iron complex are not allowed to be present in the same reagent composition, which is either the first reagent composition or the second reagent composition.
